# EUROPEAN PATENT APPLICATION

(11) **EP 3 269 365 A1**
(43) Date of publication of application: **17.01.2018**
(21) Application number: 16179431.8
(22) Date of filing: 14.07.2016
(51) Int. Cl.: A61K 31/192, A61K 31/437, A61K 31/44, A61K 31/454, A61P 35/00, A61P 35/04

(54) **KRAS INHIBITOR FOR USE IN TREATING CANCER**

(71) Applicant: Friedrich-Alexander-Universität Erlangen-Nürnberg, 91054 Erlangen (DE)
(72) Inventor: Bosserhoff, Anja-Katrin, 93053 Regensburg (DE); Dietrich, Peter, 91054 Erlangen (DE)
(74) Representative: V.O.

(57) **Abstract**

The present invention refers to inhibitors of native, non-mutated KRAS for use in preventing and/or treating malignant melanoma and/or hepatocellular carcinoma. In addition, a KRAS inhibitor is combined with an inhibitor of another factor of the Ras-Raf- MEK-ERK pathway such as a BRAF inhibitor to reach synergistic inhibitory effects and to overcome tumor cell resistance. The invention is further directed to pharmaceutical compositions comprising such inhibitor and a pharmaceutically acceptable agent.

## Description

The present invention refers to inhibitors of native, non-mutated KRAS for use in preventing and/or treating malignant melanoma and/or hepatocellular carcinoma. The invention is further directed to a pharmaceutical composition comprising such inhibitor and a pharmaceutically acceptable agent.

### Technical background

Malignant melanoma is one of the most rapidly increasing cancers worldwide (Leiter et al., 2014). Until 2011, treatment options for patients with advanced melanoma failed to improve overall survival (Olszanski, 2014). Today, targeted therapies with BRAF (V-RAF murine sarcoma viral oncogene homolog B) inhibitors, MEK (mitogen-activated protein kinase kinase) inhibitors, or immunotherapeutic approaches such as programmed death 1 (PD1) blockade improve survival of melanoma patients (Karimkhani et al., 2014). Selective BRAF inhibition (BRAFi) is the standard therapy for advanced melanoma in patients carrying BRAF mutations. 45-50% of melanoma patients harbor therapeutically attackable BRAF mutations, but the increase in progression-free and overall survival after BRAFi is modest (Luke and Ott, 2014). Several mechanisms of resistance to BRAFi were proposed (Monsma et al., 2015). One prediction for resistance to BRAF inhibitors is that mechanisms enhancing RAF dimerization result in drug resistance. These include alterations that induce RAS activity, as the canonical mechanism of RAF dimerization is RAS dependent (Lito et al., 2013). In support of this, upstream activation of RAS is associated with resistance to RAF inhibitors (Lito et al., 2013, Nazarian et al., 2010, Maertens et al., 2013). RAS isoforms play a major role in human cancer, and modern technologies have resulted in the development of promising ways to target these proteins (McCormick, 2015). Efforts to target RAS chaperone proteins have led to the development of compounds that serve as proof-of-concept molecules encouraging further attention to this newly recognized aspect of RAS signaling (McCormick, 2015, Zimmermann et al., 2013, 2013, Schmick et al., 2014, Cox et al., 2015, Stephen et al., 2014). Promising results were also achieved by systemic administration of specific siRNA targeting KRAS (McCormick, 2015, Yuan et al., 2014, Xue et al., 2014). Unlike NRAS which is mutated in melanoma in 15-20% (Posch et al., 2015), only minor attention is paid to KRAS in melanoma and hepatocellular carcinoma.

Hepatocellular carcinoma (HCC), also called malignant hepatoma, is the most common type of liver cancer. Hepatocellular carcinoma, like any other cancer, develops when there is a mutation to the cellular machinery that causes the cell to replicate at a higher rate and/or results in the cell avoiding apoptosis. In particular, chronic infections of hepatitis B and/or C can aid the development of hepatocellular carcinoma by repeatedly causing the body's own immune system to attack the liver cells, some of which are infected by the virus, others merely bystanders. While this constant cycle of damage followed by repair can lead to mistakes during repair which in turn lead to carcinogenesis, this hypothesis is more applicable, at present, to hepatitis C. Chronic hepatitis C causes HCC through the stage of cirrhosis. In chronic hepatitis B, however, the integration of the viral genome into infected cells can directly induce a non-cirrhotic liver to develop HCC. Alternatively, repeated consumption of large amounts of ethanol can have a similar effect. The toxin aflatoxin from certain *Aspergillus* species of fungus is a carcinogen and aids carcinogenesis of hepatocellular cancer by building up in the liver. Interestingly and similar to melanoma as described above, KRAS is uncommonly mutated in HCC and therefore not much recognized and unexplored as an oncogenic target yet. Therefore, to date, its functional role in HCC is elusive. Both MAPK-signaling and PI3K-signaling are prominent pathways in HCC development and progression, and it is shown in the present invention that KRAS, which can modulate both pathways, plays a crucial role in HCC. Accordingly in the context of MAPK- and PI3K-singaling, the only systemic therapy that has been shown to prolong overall survival in patients with advanced disease is treatment with Sorafenib (Schutte et al., 2014). Sorafenib is a small molecular inhibitor of intracellular tyrosine and serine/threonine protein kinases (VEGFR, PDGFR, CRAF and BRAF) that are mainly involved in MAPK- and PI3K-signaling (Mazzoccoli et al., 2015).

KRAS proteins play a major role in human cancer and have been suggested to be "undruggable" for many years. New technologies in drug discovery promoted renewed efforts to develop therapies to target RAS (McCormick, 2015). However, despite three decades of intense drug discovery efforts, no clinically feasible option for RAS inhibition has been developed. This is mostly because RAS proteins do not present suitable pockets to which drugs could bind, except for the GDP/GTP-binding site (unfortunately, RAS proteins bind very tightly to these nucleotides in picomolar affinities, with slow off-rates) (McCormick, 2015, Zimmermann et al., 2013, 2013, Schmick et al., 2014, Cox et al., 2015, Stephen et al., 2014). A number of new approaches to address RAS activity have led to the revival of this molecular target (Milroy and Ottmann, 2014, Ledford, 2015). Downstream of RAS, RAF family kinases act as primary signaling relays. The catalytic activity of RAF depends on an allosteric mechanism driven by kinase dimerization. However, RAF inhibitors can induce ERK signaling by stimulating RAF dimerization (Lavoie and Therrien, 2015, Lito et al., 2013) and small molecule inhibition of ERK dimerization could prevent tumorigenesis by RAS-ERK pathway oncogenes (Herrero et al., 2015). Importantly, RAF dimerization canonically depends on RAS activation (Villanueva et al., 2010, Richman et al., 2015, Queirolo et al., 2015). Recently, targeting KRAS processing and systemic administration of highly potent and specific siRNAs in synthetic nanoparticles were shown to inhibit KRAS driven tumors, thereby introducing intriguing possibilities for suppressing KRAS.

Promising results for prolongation of tumor free survival in melanoma patients were reported for combination of BRAF- and MEK-inhibitors (Villanueva et al., 2010, Richman et al., 2015, Queirolo et al., 2015), highlighting the importance of the MAPK-pathway (Burotto et al., 2014, Grimaldi et al., 2015). However, combination regimes with BRAF- and MEK-inhibitors are recognized to produce double resistance (Smyth et al., 2014). BRAF and MEK inhibition in metastatic melanoma can also lead to the occurrence of new KRAS-mutant carcinomas (Carlino et al., 2015), a side effect that could potentially be undermined by co-targeting KRAS. Heidron et al., (Cell, 140, 209-221, January 22, 2010) and Milagre et al. (Cancer Res., 1 July 2010, 70(13), 5549-5557) describe tumor mouse models comprising BRAF and KRAS mutations such as ^{G12D}KRAS or ^{G12V}KRAS, wherein the mutations may cooperate and according to Heidron et al. the mutations do not induce melanoma but cooperate to induce rapid onset of melanoma.

In the present invention KRAS is identified as a novel target for melanoma and/or hepatocellular carcinoma by using for example RNAi-mediated and small molecule approaches, respectivley. KRAS inhibition (KRASi) functions synergistically with another inhibitor of a factor of the Ras-Raf-MEK-ERK pathway such as an inhibitor of BRAF (BRAFi) to reduce melanoma cell proliferation and to induce apoptosis independently of BRAF mutational status. Moreover, acquired resistance to an inhibitor of a factor of the Ras-Raf-MEK-ERK pathway such as BRAFi in melanoma is dependent on dynamic regulation of KRAS expression with subsequent AKT- and ERK-activation and can be overcome by combination of KRASi and BRAFi, providing novel therapeutic regimes._Thus, the present invention focuses on the role of KRAS in progression and drug resistance of melanoma and/or hepatocellular carcinoma. Evidence is provided for the importance of wild type KRAS in melanoma and hepatocellular carcinoma, respectively, wherein KRAS is a novel therapeutic target independent of the mutational status of a factor of the Ras-Raf-MEK-ERK pathway such as BRAF. In addition, KRAS suppression, e.g. by siRNA, antibodies, small molecules etc. is effective to overcome acquired resistance to for example BRAF inhibition and co-function synergistically with selective inhibitors such as selective BRAF inhibitors.

### Summary of the invention

The present invention refers to an inhibitor of native, non-mutated KRAS for use in preventing and/or treating malignant melanoma and/or hepatocellular carcinoma. The inhibitor may not only effect KRAS but likewise mutated and/or non-mutated BRAF, wherein a mutation is homozygous or heterozygous. Inhibitors are for example selected from the group consisting of a small molecule, siRNA, antibodies and fragments thereof. In an embodiment, the KRAS inhibitor is combined with one or more other inhibitors such as an inhibitor of BRAF, EGFR and/or CRAF. The malignant melanoma and the hepatocellular carcinoma, respectively, is a primary tumor or a metastasis, which is resistant or non-resistant against an inhibitor of BRAF, EGFR and/or CRAF.

The invention further relates to a pharmaceutical composition comprising a KRAS inhibitor alone or in combination with one or more other inhibitors such as an inhibitor of BRAF, EGFR and/or CRAF. In addition, the composition optionally comprises a pharmaceutically acceptable agent which is an active or non-active agent such as an excipient, lubricants, carrier, gelating agent etc.

### Figures

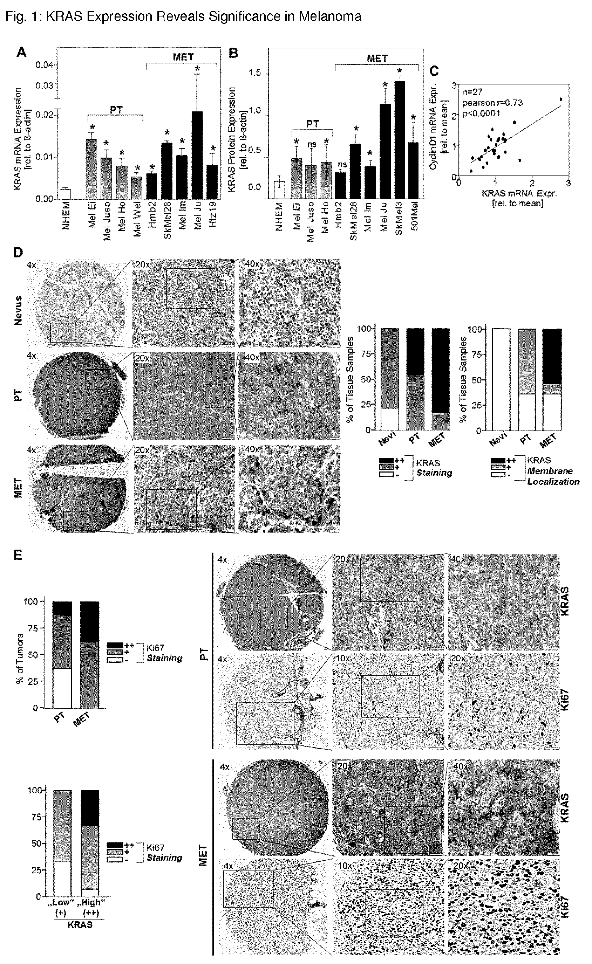

**Figure 1** **shows KRAS Expression Reveals Significance in Melanoma**
   (A and B) KRAS mRNA and protein expression levels in normal human epidermal melanocytes (NHEM, cell passage 4-5) as compared to several melanoma cell lines. (C) qRT-PCR measurement of KRAS mRNA expression (x-axis) as correlated to CyclinD1 mRNA expression in human melanoma tissue samples (r=0.73, n=27, p<0.0001). (D) KRAS staining intensity and KRAS membrane localization (exemplary images and percentage quantification) of 30 tissue micro array (TMA) samples derived from patients with either melanocyte derived benign nevi, primary melanoma, or metastatic melanoma. (E) Staining of TMAs according to (D) for Ki67 and percentage correlation to low ("+") and high ("++") KRAS staining intensity. Data are represented as mean ± SD. *: p<0.05 vs. NHEM.

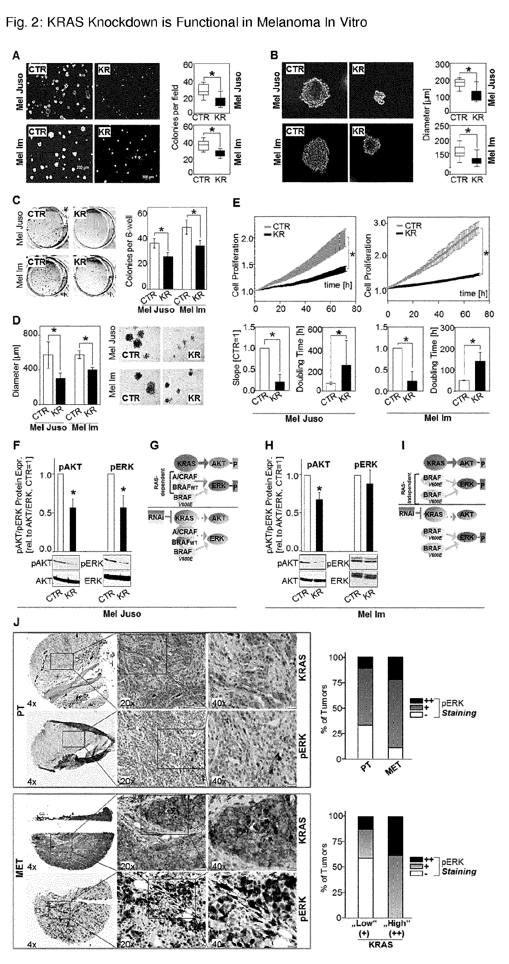

**Figure 2** **presents KRAS Knockdown is Functional in Melanoma In Vitro** (A and B) Colony number (A) and colony diameter (B) after transfection of si-CTR (CTR) or si-KRAS (KR), in three-dimensional, anchorage independent "colony forming" assays. (C and D) Colony number (C) and colony diameter (D) after transfection of si-CTR (CTR) or si-KRAS (KR), in two-dimensional, anchorage dependent "clonogenic" assays.(E) Real-time cell proliferation after transfection of si-CTR (CTR) or si-KRAS (KR). The "slope" of the curves depicting the increasing cell index and "doubling time" summarize the proliferative ability of tumor cells. (F - I) pERK/ERK and pAKT/AKT densitometric western blot analysis from Mel Juso (F) and Mel Im (H) cells after KRAS knockdown (KR) as compared to control transfected (CTR) cells and exemplary western blot images. Summary and hypothesis of the effects of RNAi-KRAS knockdown on AKT- and ERK signaling in Mel Juso (E, heterozygous ^{V600E}BRAF mutation) and Mel Im (F, homozygous ^{V600E}BRAF mutation).(J) Staining of tissue micro arrays (TMA) for pERK and percentage correlation to low ("+") and high ("++") KRAS staining intensity, according to Figure 1. Data are represented as mean ± SD in (C), (D), (E, slope and doubling time), (F), (H), and as mean ± SEM in (E, proliferation curves in *top panels).* Error bars in (A) and (B) depict the 10th and 90th percentile. *: p<0.05 vs. CTR.

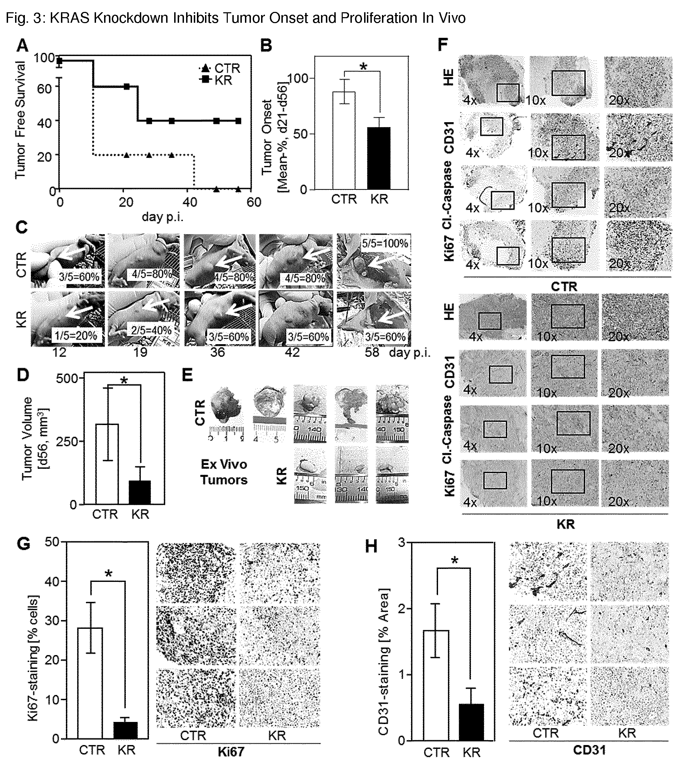

**Figure 3** **depicts KRAS Knockdown Inhibits Tumor Onset and Proliferation In Vivo**
   (A) Tumor free survival of nude mice with xenograft tumors formed after subcutaneous injection of 800.000 control transfected (CTR, n=5 mice) or si-KRAS (KR, n=5 mice) transfected Mel Im cells. (B and C) Mean tumor onset (from day 21 - day 56, depicted in percentage in B) in the KRAS Knockdown (KR) vs. control-transfected (CTR) group, and exemplary images (C). (D and E) Tumor volume at the time point of resection (day 56 post inoculation), and according images of explanted xenograft tumors. (F) Immunohistochemistry of xenograft tumor tissues (exemplary images for control=CTR vs. si-KRAS-knockdown=KR group) using antibodies against CD31, cleaved caspase 3 (Cl.-Caspase) and Ki67 as well as control staining with hematoxylin-eosin (HE) in different augmentations (4-fold, 10-fold, and 20-fold). (G and H) Ki67-(G) and CD31-immunostaining (H) of 3 xenograft tumors per group and according to densitometric quantification. Ki-67-positive cells/total cells were counted in 3 fields per tumor tissue (n=3 xenograft tumors per group) using *Image J* and confirming microscopy. CD31 was measured as percentage positive staining per area using colorimetric quantification (*Image J*). Data are represented as mean ± SD. *: p<0.05 vs. CTR.

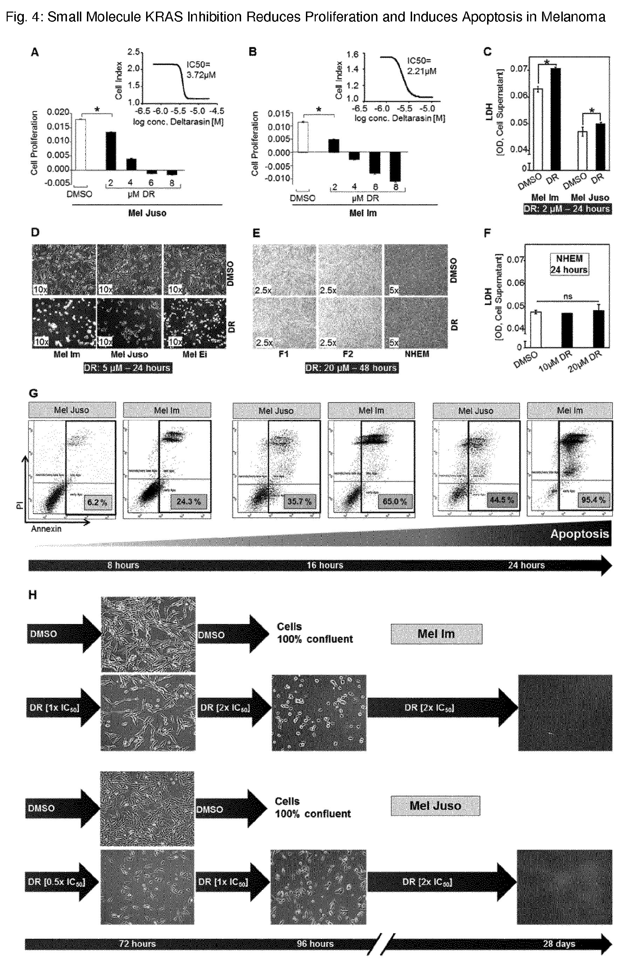

**Figure 4** **depicts small Molecule KRAS Inhibition Reduces Proliferation and Induces Apoptosis in Melanoma**
   (A and B) Cell proliferation (slope of cell index) of Mel Juso (A) and Mel Im (B) cells treated with DMSO and different concentrations of the KRAS small molecule inhibitor Deltarasin (DR). The cell index as dimensionless parameter for cell proliferative ability was used to determine the half maximal inhibitory concentration (IC50, small graphs) of DR. (C) Lactate dehydrogenase (LDH) quantification in cell supernatants (optic density, OD) after treatment with low dose DR (24 hours). (D) Exemplary images of different melanoma cell lines (Mel Im, Mel Juso and Mel Ei) treated with 5µM DR or 5µM DMSO, respectively, for 24 hours. (E and F) Images of fibroblast cell lines derived from two different donors (F1, F2) and melanocytes (NHEM) treated DR (20µM, 48 hours) or DMSO (E), and lactate dehydrogenase (LDH) quantification (F) in cell supernatants (optic density, OD) of NHEM after treatment with low dose DMSO, DR (5µM), and DR (10µM), respectively. (G) Fluorescence-activated cell sorting (FACS) analysis of apoptotic in melanoma cells in response to treatment with DR (5µM, for 8, 16, and 24 hours, respectively) in Mel Juso and Mel Im cells (exemplary images, n=3 for each time and cell line, see supplementary data). (H) Long term treatment (28 days) with DR (IC50 or 2xIC50, respectively) of Mel Im and Mel Juso melanoma cells to evaluate the emergence of surviving tumor cells and drug resistance. Data are represented as mean ± SD. *: p<0.05 vs. DMSO.

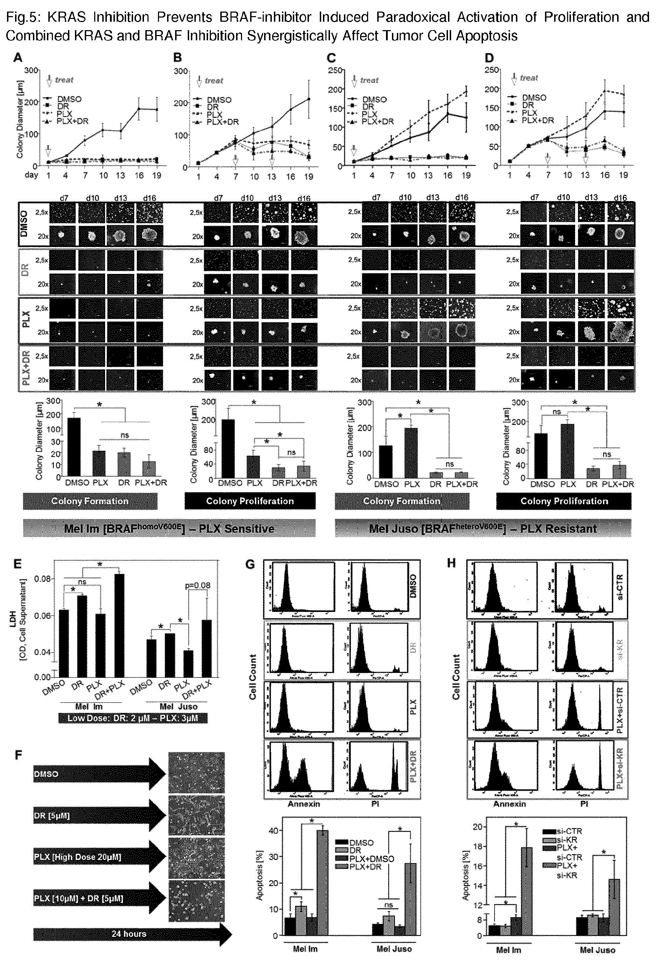

**Figure 5** **shows KRAS Inhibition Prevents BRAF-inhibitor Induced Paradoxical Activation of Proliferation and Combined KRAS and BRAF Inhibition Synergistically Affect Tumor Cell Apoptosis**
   (A - D) Time-dependent measurement of colony diameters (exemplary data in *top panels*) in three-dimensional, anchorage independent "colony forming" assays (A and B: Mel Im, C and D: Mel Juso). Exemplary Images (*middle*) and corresponding quantification of colony diameters (*bottom panels,* summarized data of n=3 experiments at day 19 after seeding) are also depicted. Treatments were performed in four different groups (10µM DMSO, 5µM Deltarasin=DR, 10µM PLX-4032=PLX (vemurafenib), or combination of 10µM PLX + 6µM DR). Effects on i) colony formation (A and C, treatment at day 1) and ii) proliferation of pre-formed colonies (B and D, treatment at day 7 and 13) were analyzed. (E) Lactate dehydrogenase (LDH) quantification in cell supernatants (optic density, OD) after treatment with DMSO, low dose DR (2 µM), PLX (3µM), or combination of DR (2µM) and PLX (3µM) for 24 hours. (F) Images of PLX-primary resistant Mel Juso cells treated with DMSO, DR (5µM), high dose PLX (20µM) and a combination of 5µM DR + 10µM PLX for 24 hours. (G) Flow cytometric (FACS) quantification of tumor cell apoptosis (percentage apoptotic cells, Mel Im and Mel Juso). Cells were treated for 5 hours with DMSO, 5µM Deltarasin (DR), 10µM PLX, or a combination of 10µM PLX and 5µM DR. According exemplary images for Annexin and propidium iodide (PI) staining are depicted for Mel Im. (H) Mel Im and Mel Juso were transfected with control si-RNA (si-CTR) or with si-KRAS (si-KR). Two subgroups (blue and red) were additionally treated with 10µM PLX (Vemurafenib) for 24 hours, with subsequent flow cytometric quantification of tumor cell apoptosis according to (G). Exemplary images for Annexin and propidium iodide (PI) staining are depicted for Mel Im. Data are represented as mean ± SD. *: p<0.05.

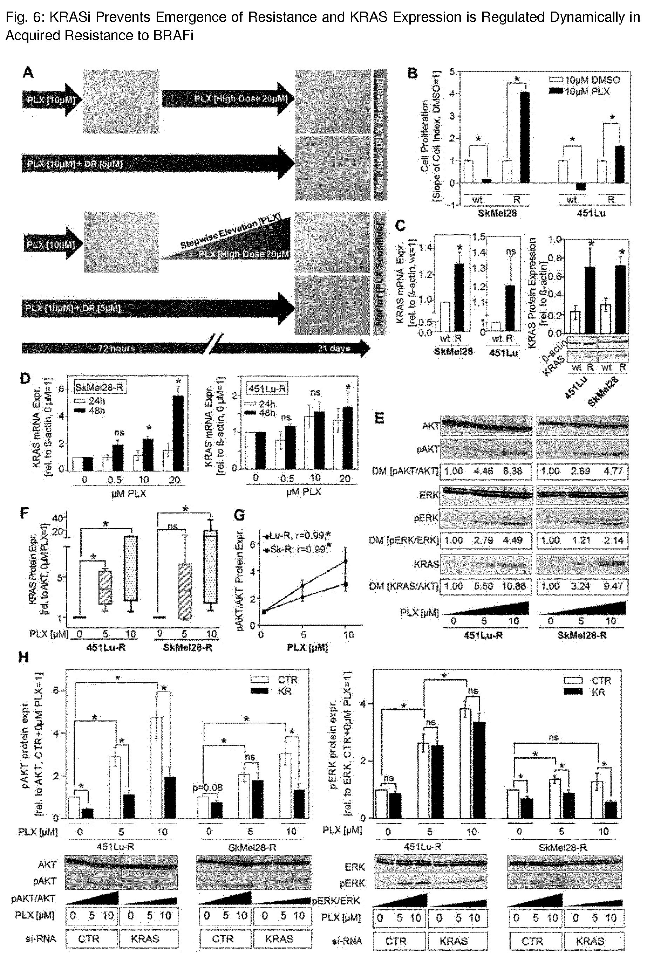

**Figure 6** **depicts KRASi Prevents Emergence of Resistance and KRAS Expression is Regulated Dynamically in Acquired Resistance to BRAFi**
   (A) Images of PLX-primary resistant Mel Juso and PLX-sensitive Mel Im cells treated for 21 days with PLX (up to 20µM) or a combination of 10µM PLX and 5µM DR.(B) Cell proliferation (slope of cell index) of PLX-sensitive (wild type=wt) and resistant (R, acquired resistance) SkMe128 and 451Lu melanoma cell lines treated with DMSO or 10µM PLX, respectively (n=2). (C) KRAS mRNA and protein expression of cell lines with acquired resistance to selective BRAF-inhibition (R, incubated with 1-5µM PLX in cell culture to remain resistant and to proliferate properly), as compared to according non-resistant (wt) cells. (D) KRAS mRNA expression of SkMe128-R and 451Lu-R cells after 24 hour- and 48 hour-treatment, respectively, with different concentrations of PLX. Asterisks indicate significant differences (p<0.05) as compared to according control (0µM PLX). (E and F) Exemplary western blot images (E) depicting immunostaining of AKT, pAKT, ERK, pERK, and KRAS. Densitometric values (DM) are also shown, representing relative quantifications. Resistant cell lines 451Lu-R and SkMe128-R were treated with 0, 5, or 10 µM PLX for 8 hours, and according summarized quantification of KRAS protein expression (n=3, F). (G) Densitometric quantification of activated AKT (pAKT/AKT) protein expression (y-axis) in dependence of PLX-doses (x-axis) in SkMe128-R and 451Lu-R cells. (H) Densitometric quantification of pAKT/AKT (*left panel*) and pERK/ERK (*right panel*) protein levels of 451Lu-R and SkMe128-R cells and exemplary western blot images. After 48 hours of transfection with either control si-RNA (CTR) or si-KRAS (KR), cells were treated with different concentrations of PLX (0 - 10µM). Data are represented as mean ± SD in A-G and as mean ± SEM in H. Ns: non-significant. *: p<0.05.

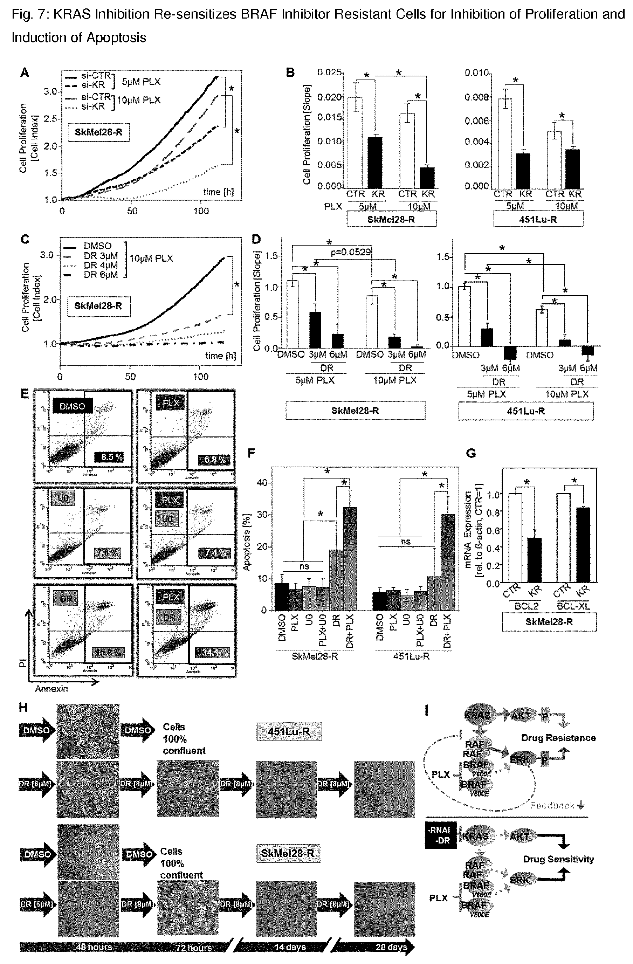

**Figure 7** **presents KRAS Inhibition Re-sensitizes BRAF Inhibitor Resistant Cells for Inhibition of Proliferation and Induction of Apoptosis**
   (A and B) Real-time cell proliferation (exemplary proliferation curve depicted in A, summarized slopes of proliferation curves depicted in B) of PLX-resistant cells treated with si-KRAS (KR) or control si-RNA (CTR), respectively, in combination with 5 or 10µM PLX. (C and D) Real-time cell proliferation (exemplary proliferation curve depicted in B, summarized slopes of proliferation curves depicted in D) of PLX-resistant cells treated with Deltarasin (DR) or DMSO, respectively, in combination with 5 or 10µM PLX, according to (A and B). (E and F) Flow cytometric analysis of apoptosis using staining for Annexin and Propidium iodide (PI) (E shows an exemplary dataset for SkMe128-R, percentage values depict apoptotic cells) and summarized quantification for both resistant cell lines (F). Treatment was performed for 24 hours with DMSO (20µM), PLX (10µM), DR (5µM), U0 (U0126=MEK1/2-inhibitor, 10µM) or combinations according to *Experimental procedures.* (G) BCL2- and BCL-XL mRNA expression as measured by qRT-PCR after KRAS knockdown (KR) in SkMe128-R melanoma cells, as compared to controls (CTR). (H) Images of long term treatment (28 days) with DR as performed in 451Lu-R and SkMe128-R cells to evaluate the emergence of surviving tumor cells and drug resistance. (I) Hypothesis for signaling effects of KRAS knockdown in acquired PLX-resistance. *Top:* ERK-induced negative feedback mechanisms on RAS-signaling are attenuated and BRAF inhibitors cause paradox transactivation of other RAF proteins, resulting in ongoing RAF- and ERK-activation (which is at least partly RAS-dependent). Apart from ERK-signaling, KRAS dependent AKT-signaling ensures survival of cancer cells during treatment with PLX. *Bottom:* Inhibition of KRAS reduces KRAS-dependent AKT- and RAF-signaling, resulting in regain of drug sensitivity. Data are represented as mean in (A and C), and as mean ± SD in (B, D, F, G). Ns: non-significant. *: p<0.05.

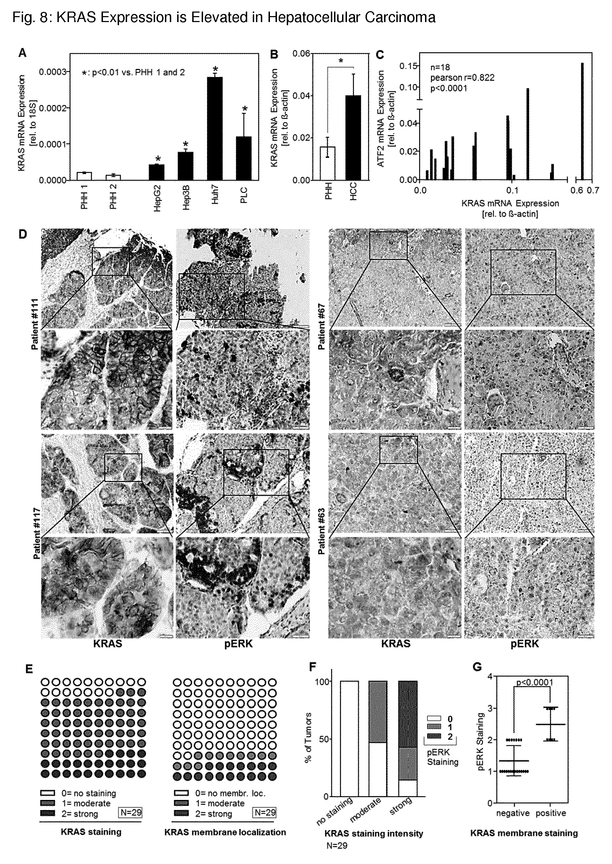

**Figure 8** **presents KRAS Expression Elevated in Hepatocellular Carcinoma**
   (A and B) KRAS mRNA expression levels in normal human hepatocytes (PHH, derived from two different donors) as compared to several HCC cell lines (A) and a cohort of 18 different patient derived HCC samples (HCC, B).
   (C) qRT-PCR measurement of KRAS mRNA expression (x-axis) as correlated to ATF2 mRNA expression in human HCC tissue samples (r=0.822, n=18, p<0.001). (D) Exemplary Images of KRAS and pERK staining of HCC tissue micro array (TMA) samples derived from patients. (E) Summary of KRAS staining of TMA samples (N=29). (F and G) Correlations of KRAS staining intensity and KRAS membrane localization with pERK staining of TMAs. Data are represented as mean ± SD. *: p<0.05 vs. PHH.

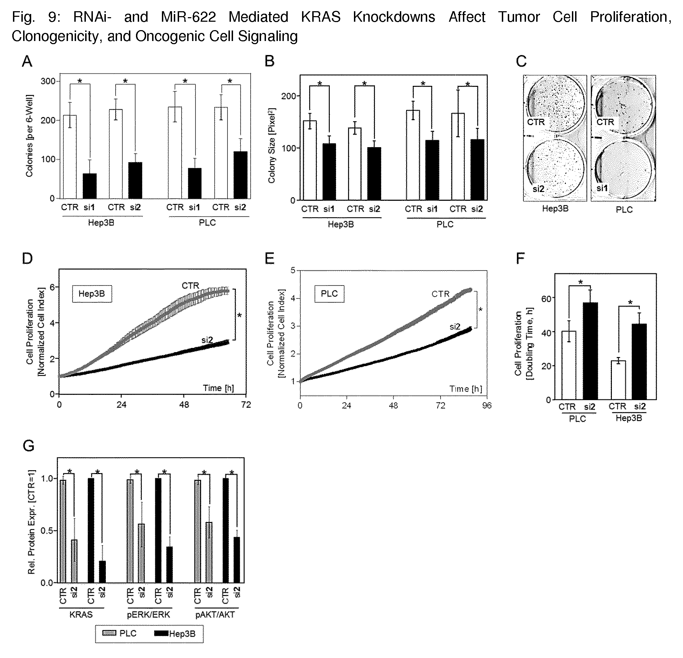

**Figure 9** **depicts RNAi- and MiR-622 Mediated KRAS KnockdownAffects Tumor Cell Proliferation, Clonogenicity, and Oncogenic Cell Signaling**
   (A - C) "Clonogenic" assay reveal anchorage-dependent colony formation and colony size in both PLC and Hep3B cells after both si**1**- and si**2**-mediated (two different siRNAs were used) KRAS knockdown.
   (D - F) KRAS knockdown effects on cell proliferation as analyzed by real-time cell proliferation for PLC and Hep3B.
   (G) Western blot analysis after KRAS knockdown showing effects on signaling of its downstream mediators, activated extracellular-signal regulated kinase (pERK) and activated v-akt murine thymoma viral oncogene (pAKT).

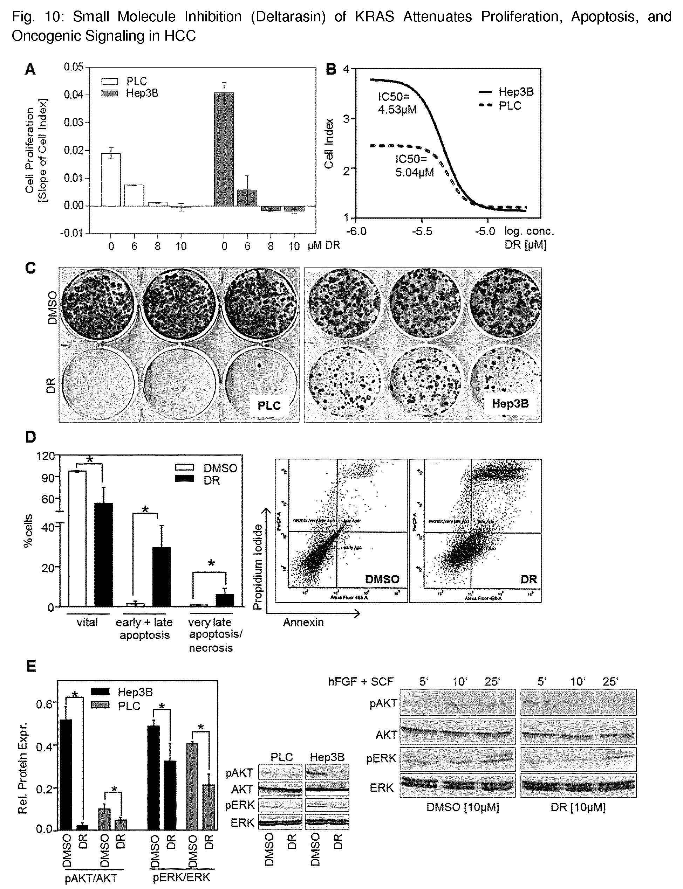

**Figure 10** **presents Small Molecule Inhibition (Deltarasin) of KRAS Attenuates Proliferation, Apoptosis and Oncogenic Signaling in HCC**
   (A and B) Deltarasin (DR) effects on dose-dependent inhibition of proliferation of both PLC and Hep3B cell lines as measured by real-time proliferation assay.
   (C) Deltarasin (6µM) effects on anchorage-dependent tumor cell growth.
   (D) Deltarasin (8µM) effects on apoptosis as measured by Annexin V and Propidium iodide (PI) staining using fluorescence-activated cell sorting (FACS) analysis (e.g. for HepG2 hepatoma cells).
   (E) Western blot analysis depicting pERK- and pAKT protein levels in PLC and Hep3B cells. Cells were treated with Deltarasin or DMSO, respectively. Moreover, activation of AKT- and ERK was measured after 5, 10, and 25 minutes of simultaneous stimulation of HCC cells using bFGF and SCF (western blot image on the right side).
**Figure 11** **shows KRAS Inhibition (Deltarasin) Enhances Chemosensitivity in Sorafenib Treated HCC cells**
   (A and B) Effects of Sorafenib (SF, 10µM) or a combinatory approach using Sorafenib and low dose (sub-lethal) KRAS inhibitor Deltarasin (DR, 5µM) on tumor cell apoptosis.
   (C) Real time cell proliferation analysis after KRAS knockdown and Sorafenib-induced inhibition of proliferation.
**Figure 12** **depicts Murine Hepatoma Cells (Hepta129) Sensitive for Small Molecule KRAS Inhibition *in vitro* and *in vivo***
   (A)Exemplary images (4-fold) depicting murine Hepatoma Cells (Hepa129) treated for 24 hours with Deltarasin (DR) and/or Sorafenib (SF) using different concentration of inhibitors.
   (B) Viable cells after treatment according to (A) as counted by microscopy.
   (C) Cell Apoptosis as measured by FACS analysis of Hepa129 cells after treatment according to (A) and (B).

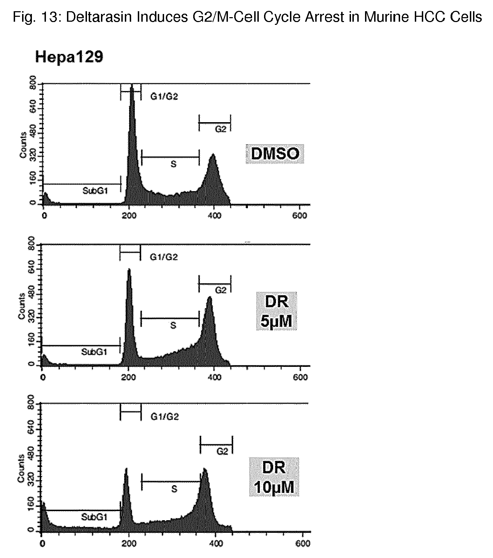

**Figure 13** **depicts Deltarasin Inducing G2/M-Cell Cycle Arrest in Murine HCC Cells**
   Fluorescence-activated cell sorting (FACS) analysis (x-axis: propidium iodide staining) reveals that 5-6 hours of Deltarasin (DR) treatment of the murine hepatoma cell line Hepa129 dose-dependently induces G2/M-Cell Cycle Arrest and increased SubG1 fractions.

### Detailed description

The present invention investigated the importance of native, non-mutated (wild type) KRAS in melanoma, and shows that KRAS is a novel therapeutic target, e.g., independent of the mutational status of another RAS and/or RAF member such as BRAF (V-RAF murine sarcoma viral oncogene homolog B). KRAS (Kirsten rat sarcoma viral oncogene homolog) expression reveals significance in melanoma *in vivo* and *in vitro.* Furthermore, KRAS knockdown reduces colony formation, colony size, and cell proliferation of melanoma cells. KRAS knockdown attenuates for example ERK (extracellular regulated MAP kinase)- and AKT (v-akt murine thymoma viral oncogene homolog 1)-signaling in, e.g., Mel Juso (primary resistance to BRAFi). In metastatic Mel Im melanoma cells (homozygous ^{V600E}BRAF- mutation, sensitive to BRAFi) for example, KRAS knockdown reduced AKT-activation. Moreover, KRAS knockdown significantly reduces tumor onset, size and staining for proliferation and angiogenesis markers of xenograft tumors. Furthermore, the PDEδ-inhibitor Deltarasin (DR) for example, which inhibits KRAS trafficking, shows significant anti-tumor effects. No evidence for toxicity for fibroblasts and melanocytes was observed.

Moreover, the present invention surprisingly shows that combined inhibition of KRAS and another factor of the Ras-Raf-MEK-ERK pathway such as KRAS and BRAF, EGFR (epidermal growth factor receptor) or CRAF inhibition results in synergistically induced tumor cell apoptosis and prevents emergence of acquired drug resistance which is the main reason for only modest progression-free survival of melanoma or HCC patients (Luke and Ott, 2014).

In the present invention in cell lines with acquired resistance to BRAF inhibitors, KRAS expression was regulated dynamically and affected MAPK- and PI3K-signaling.

This shows that acquired drug resistance to BRAFi can be mediated by dose dependent up-regulation of anti-apoptotic AKT- and pro-proliferative ERK-signaling and that these signaling pathways at least partly depend on KRAS. KRAS protein expression correlates significantly with pAKT/AKT up-regulation and KRAS knockdown reduces pERK and pAKT levels in resistant cell lines. Most interestingly since KRAS is rarely mutated in melanoma, KRAS dependent cross-talk between AKT- and ERK-signaling is shown to be independent of KRAS mutational status. Furthermore, KRAS inhibition prevents BRAF-inhibitor induced paradoxical activation of proliferation in primary resistance to BRAF inhibition.

In the present invention it is surprisingly shown that KRAS inhibition via small molecules or si-RNAs can re-sensitize inhibitor resistant cells such as BRAF, EGFR and/or CRAF inhibitor resistant cells to inhibition of proliferation and induction of apoptosis. Thus, targeting RAS or co-targeting RAS and RAF is a surprisingly successful approach for use in melanoma and/or hepatocellular carcinoma treatment. It is shown for the first time that combinatory approaches of KRASi and another factor of the Ras-Raf-MEK-ERK pathway such as BRAFi, PD-1/PD-L1 immune check point inhibitors or RAS/MAPK pathway inhibitors reveal synergism at low inhibitor doses, which does not result in resistant cells after KRAS small molecule inhibition in long term experiments. Hence, the present invention also refers to a reduction of toxic side effects which constitute further benefits of combinatory approaches.

KRAS is part of the Ras-Raf-MEK-ERK pathway which is a chain of proteins in the cell that communicates a signal from a receptor on the surface of the cell to the DNA in the nucleus of the cell. The signal starts when a signaling molecule binds to the receptor on the cell surface and ends when the DNA in the nucleus expresses a protein and produces some change in the cell, such as cell division. The pathway includes many proteins, including MAPK (mitogen-activated protein kinases, originally called ERK, extracellular signal-regulated kinases), which communicate by adding phosphate groups to a neighboring protein, which acts as an "on" or "off" switch.

An inhibitor of the present invention inhibits native, non-mutated (wildtype) KRAS either directly for example by interacting with the KRAS mRNA or protein, or indirectly by effecting the function of KRAS, e.g., by inhibiting the interaction of KRAS with another, i.e., one or more other factors of the Ras-Raf-MEK-ERK pathway. The KRAS inhibitor is used in a method of preventing and/or treating malignant melanoma and/or hepatocellular carcinoma. The inhibitor may not only inhibit KRAS alone but another factor of the Ras-Raf-MEK-ERK pathway such as BRAF, EGFR and/or CRAF in parallel. The other factor is either mutated or non mutated, and in case of a mutation the factor comprises one or more mutations in the gene. An example of such mutated factor is BRAF, e.g., ^{V600E}BRAF which is highly present in malignant melanoma patients, whereas BRAF is not or rarely mutated in hepatocellular carcinoma patients. The mutation of BRAF, EGFR, CRAF or any other factor of the Ras-Raf-MEK-ERK pathway is homozygous or heterozygous.

If KRAS and at least one additional factor of the Ras-Raf-MEK-ERK pathway such as BRAF, EGFR and/or CRAF is inhibited, all factors are directly inhibited, all factors are indirectly inhibited, or at least one factor is directly inhibited and at least one other factor is indirectly inhibited. In an embodiment of the invention the expression of KRAS and/or BRAF mRNA and/or protein, the signal transduction of KRAS and/or BRAF, and/or the trafficking of KRAS and/or BRAF is inhibited. The inhibition of KRAS alone or the inhibition of KRAS in combination with another factor of the Ras-Raf-MEK-ERK pathway such as BRAF, EGFR and/or CRAF for use in a method of preventing and/or treating of malignant melanoma and/or hepatocellular carcinoma results in induction of apoptosis and/or reduction of cell proliferation of the malignant melanoma and/or hepatocellular carcinoma. The malignant melanoma and/or the hepatocellular carcinoma is a primary tumor cell or a metastatic tumor cell.
In an embodiment of the invention the malignant melanoma and/or the hepatocellular carcinoma cell is resistant against an inhibitor of a factor of the Ras-Raf-MEK-ERK pathway. An example is a malignant melanoma and/or the hepatocellular carcinoma cell resistant against a BRAF inhibitor. As above mentioned, such resistance develops often against the standard treatment of these tumors which are BRAF inhibitors. Thus, the present invention overcomes the significant problem of resistances such as BRAF inhibitor resistance.

The KRAS inhibitor of the present invention is a small molecule, an oligonucleotide such as an antisense oligonucleotide or siRNA, an antibody or a fragment thereof. A suitable oligonucleotide of the invention is for example any oligonucleotide hybridizing with KRAS mRNA or with mRNA of any other factor of the Ras-Raf-MEK-ERK pathway which in another, e.g., a second step inhibits KRAS.

A small molecule inhibiting the expression or activity of KRAS for use in a method of preventing and/or treating malignant melanoma and/or the hepatocellular carcinoma according to the present invention is for example selected from the group consisting of compound S02 to compound S53 and compound 01 to compound 263 of WO 2014/027053 as incorporated herein by reference; inden derivatives of DE 101 63 426 as incorporated herein by reference such as Sulfindac, Ind4, Ind7, Ind9, Ind11, Ind12, mc-61, mc-231, mc-341, mc-421, mc-63, mc-233, mc-343, mc-423, mc-64, mc-234, mc-344, mc-424, mc-66, mc-236, mc-69, mc-239, mc-349, mc-429, mc-610, mc-2310, mc-3410, mc-4210, mc-611, mc-2311, mc-3411, mc-4211, mc-612, mc-2312, mc-3412, mc-4212, mc-2313, mc-3413, mc-4213, mc-615, mc-2315, mc-2317, mc-4217, mc-622, mc-2322, mc-4222, mc-623, mc-2323, mc624, mc-2324, mc-4224, EG181, EG111, EG163, EG142, EG180, EG141, EG183, EG113, EG140, EG182, EG114, EG148, EG192, EG152, EG134, EG195, EG197, EG189, EG085, EG131, EG126, EG132, EG128, EG133, EG191, EG124, EG138, EG193, EG170, EG171, EG172, EG173, EG169, EG150, EG179, EG196, EG149, EG186, EG168, EG187, EG167, EG188, EG166, EG135, EG184, EG125, EG136, EG156, EG122, EG137, EG155, EG116, EG123, EG144, EG139, EG157, and EG127; compounds 1 to 131 of WO 2015/189433 as incorporated herein by reference; and a farnesyl derivative such as salirasib (2-(((2E,6E)-3,7,11-Trimethyl-2,6,10-dodecatrienyl)sulfanyl)benzoic acid as described in WO 95/13059 and incorporated herein by reference, FTS, Farnesylthiosalicylic acid, S-Farnesylthiosalicylic acid) and any pharmaceutically acceptable salt of each of these compounds. In an embodiment of the present invention Deltarasin ((S)-1-Benzyl-2-(4-(2-(2-phenyl-1H-benzo[d]imidazol-1-yl)-2-(piperidin-4-yl)ethoxy)phenyl)-1H-benzo[d]imidazole-((S)-9)) is the KRAS inhibitor for use in a method of preventing and/or treating malignant melanoma and/or hepatocellular carcinoma.

In embodiments of the present invention the KRAS inhibitor is combined with an immunotherapy for use in a method of preventing and/or treating malignant melanoma and/or the hepatocellular carcinoma directed for example against PD-1, PD-L1 and/or CTLA4. In addition or alternatively, the KRAS inhibitor is combined with an inhibitor of BRAF such as PLX-4032 (vemurafenib) or dabrafenib, an inhibitor of EGFR such as erlotinib, and/or an inhibitor of CRAF such as sorafenib, or combinations thereof. The inhibitors are administered at the same time or at different times, the inhibitors are administered 1, 2, 3, 4, or 5 times/day, 1, 2, 3, 4, or 5 times/week, or 1, 2, 3, 4, or 5 times/month.

The present invention is further directed to a pharmaceutical composition comprising a KRAS inhibitor and a pharmaceutically acceptable agent. The agent is either therapeutically active such as an immunotherapeutic compound, a chemotherapeutic or an inhibitor of another factor of the Ras-Raf-MEK-ERK pathway, or is therapeutically inactive such as diluents, carriers, fillers, bulking agents, binders, disintegrants, disintegration inhibitors, absorption accelerators, wetting agents, lubricants, glidants, surface active agents, flavoring agents, solubility enhancers, excipient, retardant and/or gelling agent for use in preventing and/or treating malignant melanoma and/or hepatocellular carcinoma. In an embodiment of the present invention the pharmaceutical composition comprises or consists of a KRAS inhibitor and one or more compounds for use in immunotherapy of a malignant melanoma or hepatocellular carcinoma. In addition or alternatively, the pharmaceutical composition of the present invention comprises or consist of a KRAS inhibitor and an inhibitor of BRAF such as vemurafenib or dabrafenib, an inhibitor of EGFR such as erlotinib, and/or an inhibitor of CRAF such as sorenafenib, or combinations thereof.

### Examples

The following examples provide further details of the present invention but the invention is not limited to these examples.

### Example 1: KRAS Expression Reveals Significance in Melanoma

KRAS mRNA- and protein levels were up-regulated in several primary and metastatic melanoma cell lines as compared to normal human epidermal melanocytes (NHEM, cell culture passage 4-5) (Figure 1A and 1B). Next, 27 tissue samples from melanoma patients were analysed. It was found that KRAS- and CyclinD1 mRNA expression levels as measured by qRT-PCR correlated significantly (r=0.73, p<0.0001, Figure 1C). Furthermore, 30 tissue micro array (TMA) samples derived from patients with either melanocyte derived benign nevi, primary melanoma, or metastatic melanoma were compared. Both KRAS staining intensity and KRAS membrane localization were elevated in melanoma tissues as compared to nevi. The strongest KRAS staining intensity and membrane localization was detected in metastatic tissues (Figure 1D). Co-staining for Ki67 showed enhanced Ki67-expression in metastatic as compared to primary melanoma tissues, and samples with high KRAS expression displayed stronger Ki67 staining as compared to samples with low KRAS expression (Figure 1E). Together, *in vitro* and *in vivo* analysis of KRAS expression point to significance in melanoma.

### Example 2: KRAS Knockdown is Functional in Melanoma In Vitro

In the following, it was focused on the functional role of KRAS in melanoma using metastatic as well as primary melanoma cells. These cell lines carry no mutations in the KRAS or NRAS gene locus. KRAS was suppressed by RNA-Interference (RNAi).

Two different approaches of RNAi were used (single si-RNAs and si-RNA-Pools). KRAS knockdown (KR) significantly reduced anchorage-independent and anchorage-dependent tumor colony formation and colony size as compared to mock transfected control (CTR) cells (Figure 2A - 2D). Moreover, KRAS knockdown caused sustained reduction of tumor cell proliferation (Figure 2E). In both Mel Juso (primary melanoma cell line, heterozygous ^{V600E}BRAF-mutation) and Mel Im (metastatic melanoma cell line, homozygous ^{V600E}BRAF-mutation), KRAS knockdown significantly attenuated signaling of its downstream mediator activated v-akt murine thymoma viral oncogene (pAKT). Moreover, KRAS knockdown suppressed activated extracellular-signal regulated kinase (pERK) significantly in Mel Juso cells but not in Mel Im cells. This is consistent with the finding that homozygous ^{V600E}BRAF-mutations (Mel Im) lead to RAS-independent ERK activation (Figure 2F - 2I). Tissue micro array analysis revealed stronger pERK staining in patient tissues with high KRAS staining as compared to samples with low KRAS staining (Figure 2J). In summary, KRAS knockdown reduces colony formation, colony size, and tumor cell proliferation and affects oncogenic MAPK- and PI3K/AKT-signaling in melanoma.

### Example 3: KRAS Knockdown Inhibits Tumor Onset and Proliferation In Vivo

To investigate KRAS knockdown effects on melanoma cells *in vivo,* Mel Im with si-RNA suppressed KRAS and controls, respectively, were subcutaneously injected into *nu*/*nu* mice to form xenograft tumors. During observation, body weight was stable in both groups. Tumor free survival was enhanced (Figure 3A), and tumor onset was reduced (Figure 3B and 3C) in the KRAS knockdown group, respectively, as compared to controls. The tumor volume of explanted xenograft tumors was also reduced in the KRAS knockdown group (Figure 3D and 3E). Immunohistochemistry showed reduced staining for the proliferation marker Ki67 in the KRAS knockdown group (Figure 3F and 3G). The macroscopic impression of reduced vasculature of explanted xenograft tumors in the KRAS knockdown group was confirmed by staining of the endothelial cell marker CD31 (Figure 3H). Both Ki67 and CD31 expression are known independent prognostic markers for melanoma patients (Gimotty et al., 2005, Depasquale and Thompson, 2005). Together, these data support the marked *in vitro* effects and reveal KRAS as an oncogenic target for melanoma *in vivo.*

### Example 4: Small Molecule KRAS Inhibition Reduces Proliferation and Induces Apoptosis in Melanoma

The marked effects of KRAS knockdown raised the question if pharmacologic inhibition of KRAS could be an effective approach. By now, all attempts to target KRAS failed in clinical studies (McCormick, 2015). Recently, a small-molecule inhibitor - "Deltarasin" (DR) - was identified. Deltarasin binds to the delta subunit of rod-specific photoreceptor phosphodiesterase (PDEδ), a protein that regulates the trafficking of KRAS between membranes. DR was shown to reduce proliferation of pancreatic adenocarcinoma cells (Zimmermann et al., 2013), but has not been used in melanoma studies before. In the present experiments, DR caused dose-dependent inhibition of proliferation of Mel Juso (Figure 4A) and Mel Im (Figure 4B) cells. Treatment with low dose DR (2µM) was sufficient to increase lactate dehydrogenase (LDH) amounts in cell supernatants (Figure 4C). Moderate doses of DR (5µM) showed marked toxicity in different melanoma cell lines after 24 hours (Figure 4D). However, doses up to 20µM DR showed no toxic effects in human fibroblast cell lines and in normal human epidermal melanocytes (NHEM) (Figure 4E and 4F). Fluorescence-activated cell sorting (FACS) analysis revealed marked time-dependent induction of apoptosis in response to DR-treatment (Figure 4G). Moreover, to evaluate the possible emergence of surviving tumor cells and drug resistance in response to DR, long term treatment with increasing doses was performed. After 28 days, no surviving tumor cells were detected after DR-treatment (Figure 4H). Together, these results demonstrate strong anti-tumor effects on melanoma cells by using small molecule KRAS inhibition.

### Example 5: KRAS Inhibition Prevents BRAF-inhibitor Induced Paradoxical Activation of Proliferation and Combined KRAS and BRAF- Inhibition Synergistically Affect Tumor Cell Apoptosis

BRAF inhibition (BRAFi) is one of the few clinical approaches with modest survival benefits for metastasized melanoma patients carrying BRAF mutations (Karimkhani et al., 2014, Luke and Ott, 2014). Combination regimens have been developed to further delay the onset and to break acquired resistance. Combined BRAF and MEK inhibition improves survival as compared to single-agent approaches and has now received regulatory approval, giving evidence of the importance of the MAPK pathway in melanoma therapy (Johnson and Sosman, 2015, Burotto et al., 2014). Here, a novel approach combining BRAFi with inhibition its *upstream* mediator KRAS was tested. For BRAFi, the clinically approved standard drug PLX-4032 ("Vemurafenib", referred to as "PLX") was used. DR (5µM) and PLX (10µM) effects on three-dimensional colony formation as well as on proliferation of pre-formed colonies were analyzed. In BRAFi primary sensitive Mel Im and Htz19 melanoma cells, DR was similarly effective as PLX and the DR+PLX combination resulted in almost complete abolishment of colony formation and proliferation (Figure 5A and 5B). In BRAFi primary resistant Mel Juso cells, PLX enhanced ERK-activation, colony formation and colony proliferation (Figure 5C and 5D), most likely due to "paradox" transactivation of wild type BRAF proteins (Lito et al., 2013, Sondergaard et al., 2010). However, in Mel Juso, DR markedly inhibited colony formation and proliferation (Figure 5C and 5D). Noteworthy, this effect was also present when DR was combined with PLX, thereby completely abolishing the paradoxical growth-enhancing effects of PLX in this primary BRAFi resistant melanoma cells. Analyzing tumor cell apoptosis, we found that compared to single agent treatment, a combinatory approach using low inhibitor doses (2µM DR, 3µM PLX) was sufficient to increase lactate dehydrogenase (LDH) amounts in Mel Im cell supernatants (Figure 5E). PLX primary resistant Mel Juso cells were treated with a combination of moderate doses of PLX (10µM) and DR (5µM), which revealed marked tumor cell toxicity as compared to single agent treatments (Figure 5F). Therefore, in the following, short treatment intervals and moderate doses of DR were used to avoid/reduce single agent related apoptosis. FACS analysis revealed that five hours of treatment with 5µM DR only slightly induced apoptosis in Mel Im (Figure 5G) and Htz19 and did not induce apoptosis in Mel Juso (Figure 5G). Treatment with 10µM PLX for 5 hours was also insufficient to induce apoptosis (Figure 5G). However, the combination of DR+PLX significantly enhanced apoptosis as compared to single agents. Subsequently, a second approach to confirm synergism of combined KRASi and BRAFi by using si-RNA was performed. Here, after 24 hours, PLX slightly enhanced apoptosis in PLX-sensitive Mel Im and Htz19 and did not cause apoptosis in Mel Juso cells (Figure 5H). The combination of RNAi-mediated KRAS knockdown and PLX enhanced apoptosis in all three cell lines, supporting the above described synergistic effects (Figure 5H). Together, these data show that KRASi prevents BRAF-inhibitor induced paradoxical activation of proliferation and reveal synergistic effects of combined BRAFi and KRASi on tumor cell apoptosis.

### Example 6: KRASi Prevents Emergence of Drug Resistance and KRAS Expression is Regulated Dynamically and Affects MAPK- and PI3K-Signaling in Acquired Resistance to BRAFi

After treatment with increasing doses of PLX, BRAF inhibitor sensitive Mel Im reveal survival of a moderate proportion of tumor cells. This confirms the common finding of rapid development of *acquired* resistance to BRAFi as reported in numerous studies. However, the combination of 5µM DR and 10µM PLX was sufficient to completely abolish the emergence of surviving tumor cells (Figure 6A). In the present example two pairs of both BRAFi-sensitive (-wt) and BRAFi-resistant (-R) ^{V600E}BRAF melanoma cell lines (451Lu and SkMe128) were used. The resistant cell lines reveal acquired drug resistance and exhibit cross-resistance to different BRAF inhibitors, including PLX (Villanueva et al., 2010). The cell lines do not reveal secondary BRAF mutations beyond V600E or *de novo* mutations or copy number alterations in NRAS, C-KIT and PTEN (Villanueva et al., 2010). PLX inhibited proliferation in SkMe128-wt and 451Lu-wt, whereas cell proliferation in both resistant cell clones was enhanced in response to treatment with PLX (Figure 6B). Interestingly, it was found that KRAS mRNA and protein expressions were elevated in resistant cell lines as compared to non-resistant cell lines (Figure 6C). Noteworthy, the resistant cell clones were continuously incubated with PLX to remain acquired drug resistance and to proliferate properly, pointing to a possible effect of PLX treatment on KRAS expression. Accordingly, qRT-PCR analysis revealed dose-dependent slight dynamic alteration of KRAS mRNA expression in resistant and also some non-resistant melanoma cell clones in response to PLX treatment (Figure 6D). Moreover, western blot analysis confirmed that eight hours of PLX treatment dose-dependently enhanced KRAS protein expression in resistant cell lines (Figure 6E and 6F). Furthermore, treatment with PLX dose-dependently increased the activation of AKT (Figure 6G). EGFR induced PI3K/AKT pathway activation is known to be crucial for BRAF inhibitor resistance (Wang et al., 2015, Gross et al., 2015), and dynamic RAF kinase switch induces resistance that could be overcome by co-targeting MEK- and IG-1R-dependent PI3K-signaling (Villanueva et al., 2010). However, next to EGFR and IG-1-R, KRAS is known to activate the PI3K/AKT pathway. Therefore, the present data point to anintrinsic mechanism of AKT-activation in BRAFi resistance which is mediated by KRAS. Accordingly, it was found that KRAS knockdown was sufficient to inhibit PLX-induced increased AKT activation in both resistant cell lines (Figure 6H). Furthermore, resistant cell lines showed dose-dependent "paradoxical" increased ERK activation when treated with PLX (Figure 6E). However, PLX-induced increased ERK activation was significantly reduced after KRAS knockdown in SkMe128-R (Figure 6H).

### Example 7: KRAS Inhibition Re-sensitizes BRAFi Resistant Cells for Inhibition of Proliferation and Induction of Apoptosis

Subsequently, KRAS knockdown markedly reduced proliferation in PLX-treated, BRAFi-resistant melanoma cells (Figure 7A and 7B). KRAS knockdown led to enhanced inhibition of proliferation in SkMe128-R that were treated with higher doses of PLX as compared to lower doses. Indeed, inhibition of proliferation was ~2.5-fold stronger in SkMe128-R treated with 10µM PLX *versus* 5µM. In accordance to the dynamic KRAS mRNA and protein alterations in response to PLX, these data support that resistant cell clones could dynamically react within (K)RAS signaling in response to increasing PLX doses, making them more dependent on KRAS signaling. Moreover, small molecule KRAS inhibition (DR) led to dose dependent inhibition of proliferation (Figure 7C and 7D). Subsequently, analyzing apoptosis, it was found that the resistant cell lines showed cross-resistance to MEK- and even combined BRAF-/MEK-inhibition. Combining DR and PLX exerted synergistic apoptotic effects in resistant cell lines (Figure 7E and 7F). Accordingly, using SkMe128-R, RNAi-mediated KRAS knockdown confirmed increased apoptosis in response to BRAFi already after 6 hours. Moreover, KRAS knockdown led to reduction of anti-apoptotic BCL-XL and/or BCL2 mRNA expression, respectively, in SkMe128-R, 451Lu-R, Mel Im and Mel Juso (Figure 7G). Both ERK- and AKT-signaling are known to promote transcription of apoptosis-inhibiting genes like BCL2, BCL-XL and MCL1 (Sale and Cook, 2013, Wilson et al., 1996, Boucher et al., 2000, Yang et al., 2015). Thus, our data point out that AKT- and ERK-signaling affect transcriptional regulation of apoptosis related genes in acquired BRAFi resistance in melanoma and support the apoptosis "sensitizing" effect of KRAS inhibition. Finally, the emergence of cross-resistance to DR in PLX-resistant cell lines was examined, and found that 8µM DR was sufficient to completely abolish the emergence of surviving tumor cells over a sustained period (Figure 7H). Together, the results show that acquired drug resistance to BRAFi is mediated by dose-dependent dynamic KRAS expression affecting anti-apoptotic AKT- and pro-proliferative ERK-signaling. Furthermore, RNAi-mediated- and small molecule KRASi can re-sensitize resistant cell lines for PLX treatment (Figure 7I).

### Example 8: KRAS Expression Reveals Significance in HCC

KRAS is uncommonly mutated in HCC and therefore not much recognized and unexplored as an oncogenic target, and its functional role in HCC is elusive. Thus, the role of KRAS in HCC was investigated. First, qRT-PCR analysis revealed that KRAS mRNA levels are significantly upregulated in HCC cells as compared to primary human hepatocytes (PHH) (Figure 8A). A subsequent screen of 14 different pairs of HCC samples showed upregulation of KRAS mRNA levels in HCC as compared to primary human hepatocytes (PHH) (Figure 8B). Moreover, analysis of tissue micro array (TMA) with patient derived HCC samples revealed significant expression of KRAS protein and correlation of KRAS staining with pERK expression levels (Figure 8E-8G).

### Example 9: RNAi-mediated KRAS Knockdown Inhibits Tumor Cell Proliferation and Affects Oncogenic Signaling in HCC

Examining the functional role of KRAS in HCC, next RNAi-mediated knockdown experiments using a commercially available si-RNA against KRAS (si**1**). Moreover, for confirmation of target specificity, another approach of specific RNAi-mediated KRAS knockdown using a "si-POOL" containing a complex pool of 30 selected siRNAs was performed, each targeting the KRAS mRNA. Si-RNA-Pools are believed to reduce off-target effects and to have sustained efficiency as compared to single si-RNAs. qRT-PCR analysis was performed to validate significant knockdown of KRAS mRNA levels after si**1**- or si**2**-transfection in PLC and Hep3B (data not shown). Subsequent "clonogenic" assays revealed strong suppression of anchorage-dependent colony formation and colony size in both PLC and Hep3B cells after both si**1**- and si**2-**mediated KRAS knockdown (Figure 9A - 9C). These effects were confirmed in a third HCC cell line (HepG2) after KRAS knockdown by si**2** (data not shown). Furthermore, KRAS knockdown caused sustained strong reduction of cell proliferation in a real-time cell proliferation assay in PLC and Hep3B (Figure 9D - 9F). Subsequent western blot analysis showed that KRAS knockdown by si**2** significantly attenuated signaling of its downstream mediators, activated extracellular-signal regulated kinase (pERK) and activated v-akt murine thymoma viral oncogene (pAKT) (Figure 9G). Together, KRAS knockdown experiments show strong tumor suppressive function and si-RNA-mediated KRAS inhibition is sufficient to significantly reduce oncogenic MAPK- and PI3K-signaling in HCC.

### Example 10: Small Molecule Inhibition of KRAS Affects Proliferation, Apoptosis, and Oncogenic Signaling in HCC

The strong tumor suppressive effects of KRAS knockdown raised the question if pharmacologic inhibition of KRAS could be an effective approach in HCC treatment. By now, all attempts to target KRAS failed in clinical studies (McCormick, 2015). Recently, a small-molecule inhibitor - "Deltarasin" (referred to as "DR") - was identified. DR binds to the delta subunit of rod-specific photoreceptor phosphodiesterase (PDEδ), a protein that regulates the trafficking of KRAS to membrane compartment (Zimmermann et al., 2013). However, DR was never used before in HCC. In our study, DR caused strong dose-dependent inhibition of proliferation of both PLC and Hep3B cell lines as measured by real-time proliferation assay (Figure 10A and 10B) and low doses suppressed anchorage-dependent tumor cell growth (Figure 10C). Higher doses of DR (>8µM) showed marked tumor cell toxicity, and Annexin V and Propidium iodide (PI) staining with subsequent fluorescence-activated cell sorting (FACS) analysis revealed marked dose-dependent induction of apoptosis (e.g. for HepG2 hepatoma cells, Figure10D). Moreover, western blot analysis revealed significant reduction of pERK- and pAKT protein levels in PLC and Hep3B cells, giving evidence that accordingly to si-RNA-mediated KRAS knockdown, pharmacologic inhibition of KRAS signaling in HCC is sufficient to inhibit MAPK- and PI3K-singaling (Figure 10E). Accordingly in the context of MAPK- and PI3K-singaling, the only systemic therapy that has been shown to prolong overall survival in patients with advanced disease is treatment with Sorafenib (Schutte et al., 2014).

### Example 11: KRAS Inhibition Enhances Sorafenib Effects in HCC Cells

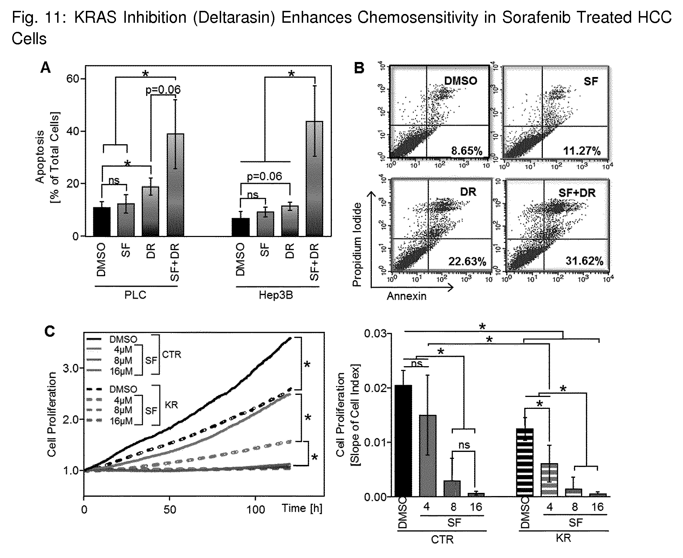

Therefore, in the following, the question was addressed if combinatory approaches using KRAS inhibition together with Sorafenib could be effective for HCC treatment. The effects of Sorafenib (SF, 10µM) or a combinatory approach using Sorafenib and low dose (sub-lethal) KRAS inhibitor Deltarasin (DR, 5µM) on tumor cell apoptosis were determined. Here, it was found that Sorafenib and Deltarasin can function synergistically to induce tumor cell apoptosis in PLC and Hep3B HCC cells (Figure 11A and 11B). Moreover, real time cell proliferation analysis revealed that that after KRAS knockdown (using si**2**), Sorafenib-induced inhibition of proliferation was enhanced markedly and dose-dependently (Figure 11C). Since it was found that KRAS suppression affects MAPK- and PI3K-signaling pathways in HCC (see above), these data support the hypothesis that the combination of Sorafenib and pharmacologic or si-RNA-mediated KRAS inhibition could be an effective therapeutic approach in advanced HCC.

### Example 12: KRAS Inhibition using the Small Molecule Inhibitor Deltarasin to induce G2/M-Cell Cycle Arrest in HCC Cells

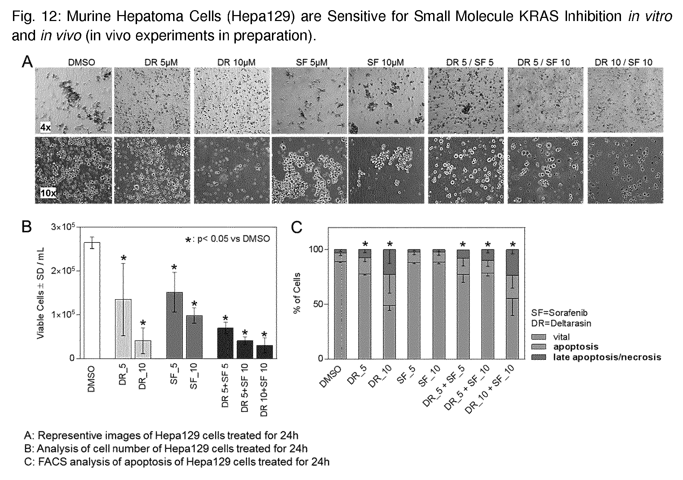

Further, the effects of Deltarasin on the murine hepatoma cell line "Hepa129" was analysed. In accordance to human hepatocellular carcinoma cell lines, Deltarasin exerts marked effects on tumor cell proliferation (Figure 12A and 12B) and apoptosis (Figure 12C). Interestingly, fluorescence-activated cell sorting (FACS) analysis revealed that 5-6 hours of Deltarasin (DR) treatment of the murine hepatoma cell line Hepa129 dose-dependently induces G2/M-Cell Cycle Arrest and increased SubG1 fractions (Figure 13). This shows a possible novel mechanism of action for induction of apoptosis in HCC cells mediated by the small molecule KRAS inhibitor Deltarasin, i.e. induction of a G2/M-Cell cycle arrest finally resulting in apoptosis.

## Claims

1. Inhibitor of native, non-mutated KRAS for use in a method of preventing and/or treating malignant melanoma and/or hepatocellular carcinoma.

2. Inhibitor for use according to claim 1, further inhibiting mutated and/or non-mutated BRAF.

3. Inhibitor for use according to claim 1 or 2, wherein the expression of KRAS and/or BRAF mRNA and/or protein, the signal transduction of KRAS and/or BRAF, and/or the trafficking of KRAS and/or BRAF is inhibited.

4. Inhibitor for use according to any one of claims 1 to 3, wherein apoptosis is induced and/or cell proliferation of the malignant melanoma and/or hepatocellular carcinoma is reduced.

5. Inhibitor for use according to any one of claims 1 to 4, wherein a mutation of BRAF is homozygous or heterozygous.

6. Inhibitor for use according to any one of claims 2 to 5, wherein the BRAF mutation is ^{V600E}BRAF.

7. Inhibitor for use according to any one of claims 1 to 6, wherein the malignant melanoma and/or the hepatocellular carcinoma is a primary tumor cell or a metastatic tumor cell.

8. Inhibitor for use according to any one of claims 1 to 7, wherein the malignant melanoma and/or the hepatocellular carcinoma is resistant against a BRAF inhibitor.

9. Inhibitor for use according to any one of claims 1 to 8, wherein the inhibitor is a small molecule and/or an oligonucleotide such as an antisense oligonucleotide or siRNA.

10. Inhibitor for use according to claim 9, wherein the small molecule is Deltarasin or salirasib.

11. Inhibitor for use according to any one of claims 1 to 10, wherein the inhibitor is combined with a malignant melanoma or hepatocellular carcinoma immunotherapy.

12. Inhibitor for use according to any one of claims 1 to 11, wherein the inhibitor is combined with an inhibitor of BRAF such as PLX-4032 (vemurafenib) or dabrafenib, an inhibitor of EGFR such as erlotinib, and/or an inhibitor of CRAF such as sorafenib, or combinations thereof.

13. Pharmaceutical composition comprising an inhibitor according to any one of claims 1 to 10 and a pharmaceutically acceptable agent for use in a method of preventing and/or treating malignant melanoma and/or hepatocellular carcinoma.

14. Pharmaceutical composition according to claim 13, further comprising one or more compounds for a malignant melanoma or hepatocellular carcinoma immunotherapy.

15. Pharmaceutical composition according to claim 13 or 14, further comprising an inhibitor of BRAF, EGFR and/or CRAF.
